(19) Europäisches Patentamt
European Patent Office
Office européen des brevets

(11) Veröffentlichungsnummer: **0 026 539**
**B2**

# (12) NEUE EUROPÄISCHE PATENTSCHRIFT

(45) Veröffentlichungstag der neuen Patentschrift: **18.10.89**

(51) Int. Cl.⁴: **A 61 K 7/16, A 61 K 7/22**

(21) Anmeldenummer: **80200901.9**

(22) Anmeldetag: **25.09.80**

## (54) Orale Kompositionen mit stabilisierten Zinnsalzen.

(30) Priorität: **02.10.79 GB 7934159**
**24.01.80 GB 8002433**
**01.02.80 GB 8003525**

(43) Veröffentlichungstag der Anmeldung
**08.04.81 Patentblatt 81/14**

(45) Bekanntmachung des Hinweises auf die Patenterteilung
**02.01.85 Patentblatt 85/1**

(45) Bekanntmachung des Hinweises auf die Entscheidung über den Einspruch:
**18.10.89 Patentblatt 89/42**

(84) Benannte Vertragsstaaten:
**AT BE CH DE FR GB IT LI LU NL SE**

(73) Patentinhaber: **Gaba International AG, St. Johannsvorstadt 98, CH-4004 Basel (CH)**

(72) Erfinder: **Mühlemann, Hans-Rudolf, Prof. Dr. med., Beustweg 8, CH-8032 Zürich (CH)**
Erfinder: **Schmid, Hans, Dipl.-Chem., Andlauerstrasse 4, CH-4132 Muttenz (CH)**

(74) Vertreter: **Frossard, Michel, Dr. et al, A. Braun, Braun, Héritier, Eschmann AG, Patentanwälte Holbeinstrasse 36-38, CH-4051 Basel (CH)**

(56) Entgegenhaltungen:
**BE-A- 569 397**
**CA-A- 594 553**
**DE-A- 2 523 363**
**DE-A- 2 755 847**
**FR-A- 2 374 902**
**US-A- 3 083 143**
**US-A- 3 235 459**
**US-A- 3 544 637**
**US-A- 3 932 604**
**US-A- 4 117 109**
**US-A- 4 157 386**

**CHEMICAL ABSTRACTS, Band 78, Heft 26,2Juli 1973, Seite 280, Zusammenfassung 163957s, COLUMBUS OHIO (US)**
**Accepted Dental Therapeutics, 39. Auflage, Herausgeber Amer. Dent. Assoc., Chicago (IL, USA) 1982, 356-360**
**I.L. Shannon, J. Southern Calif. State Dent. Assoc. 32 (1964), 67-70**

(56) Entgegenhaltungen: (Fortsetzung)
**J.K. Lim, J. Dent. Res. 49 (1970), 760-767**
**P.E. Norris, J. Amer. Pharm. Assoc. 20 (1959), 86-87**
**I.L. Shannon u. Mitarb., J. Southern Calif. State Dent. Assoc. 33 (1965), 520-522**
**I.L. Shannon, Caries Res. 3 (1969), 339-437**
**J.K. Lim, J. Dent. Res. 50 (1971), 531-535**
**J. E. ELLINGSEN et al., Acta. Odontal. Scand., 38 (1980), S. 219-222**
**S.L. YANKELL et al., J. Perisdont. Res. 17 (1982), S: 374-379**
**Accepted Dental Therapeutics, 39. Auflage, Library of Congress, Chicago (Illinois, USA), (1982), S. 356-357**
**J.R. Mellberg et al., "Fluoride in Reventive Dentistry', Quintessence Publishing Co. Inc., Chicago, (Il. USA) (1983), S. 154, 155, 184**

**Die Akte enthält technische Angaben, die nach dem Eingang der Anmeldung eingereicht wurden und die nicht in dieser Patentschrift enthalten sind.**

EP 0 026 539 B2

**Beschreibung**

Gegenstand der Erfindung sind orale Kompositionen zur Vorbeugung und Bekämpfung der Plaquebildung und der Zahnkaries, welche Zinn(II)salze in einem wasserhaltigen Medium enthalten und deren Beständigkeit über eine längere Zeitdauer durch Stabilisierung der Zinnsalze gewährleistet ist. Unter die Bezeichnung orale Kompositionen fallen insbesondere Zahnpasten, Gelées, Mundwasser, Mundsprays, Prophylaxepasten, Lösungen zur Lokalapplikation und dergleichen.

Die Zinn(II)-ionen haben eine plaquehemmende Wirkung; sie ist am Hund bei lokaler Anwendung und am Mensch bei Verwendung von $SnF_2$ enthaltenden Mundwassern nachgewiesen worden. Es wurde auch festgestellt, dass Mundspülungen mit $SnF_2$ in einer Konzentration an $Sn^{++}$ von 780 bzw. 3100 ppm die Bildung von Säure aus Saccharose in den Bakterienablagerungen herabsetzen. $SnF_2$ ist als Mittel gegen die Zahnkaries von der Wissenschaft allgemein anerkannt und wirksamer Bestandteil karieshemmender Zubereitungen.

So wird in der Europäischen Patentanmeldung 11663 (Unilever Ltd.) eine Zubereitung für die Mund- und Zahnhygiene beschrieben, welche Tetradecylamin, eine Zink-, Aluminium-, Kupfer-, Eisen-, Nickel-, Kobalt- oder Zinnverbindung und eine Base enthält und deren erstgenannten Komponenten eine synergistische Hemmwirkung auf die Bildung der Plaque ausüben. In der US-Patentschrift 4 117 109 (G.K. Stookey; Indiana University Foundation) wird eine Zahnpaste vorgeschlagen, welche als Schleifmittel Calciumpyrophosphat, als Füllstoff Siliciumdioxid, ein oberflächenaktives Mittel und gegebenenfalls als Antikariesmittel Natriumfluorid enthält; sie bewirkt eine gute Reinigung der Zähne bei geringerer Schleifwirkung. Gegenstand des US-Patentes 3 932 604 (J.B. Barth; Colgate-Palmolive Co.) ist eine Dentalcrème mit einem Mindestgehalt von 15 Gew.-% an Xylitol als Anfeuchtungsmittel, wodurch die Behälteröffnung, wenn offen belassen, sich nicht verstopft; das Xylitol wirkt zudem als Süssstoff, ohne Karies-fördernd zu sein. Die Crème kann ferner u.a. Natriumfluorid, Zinn(II)-fluorid, Schleifmittel und oberflächenaktive Mittel, wie das Natriumsalz von sulfonierten höheren Fettsäuren, Polyäthylenoxid-sorbitanmonostearat usw., enthalten. In der US-Patentschrift 4 157 386 (P.J. La Rochelle) wird eine Kaupastille aus einem Anfeuchtungsmittel wie Xylitol, einem Schleifmittel, einem Klebstoff aus Stärke und zur Einstellung der Viskosität einem ungesättigten pflanzlichen Öl beschrieben. Wird sie in den Mund genommen, so bildet sie mit dem Speichel einen klebenden Überzug auf den Zähnen, der nur durch Bürsten entfernt werden kann. Die Pastille kann auch Natriumfluorid, Zinn(II)-fluorid, Schleifmittel und oberflächenaktive Mittel enthalten.

In früheren Patentschriften der Anmelderin (Belgisches Patent 569 397, US-Patent 3 083 143 und Deutsche Auslegeschrift 1 198 493 – H. Schmid und H.R. Mühlemann; Gaba AG) wird die Karies-hemmende Wirkung der Hydrofluoride organischer Amine beschrieben; sie unterdrücken nämlich die auflösende Wirkung der Säuren auf den Zahnschmelz. Eine ebenfalls ausgeprägte Hemmwirkung gegenüber der Karies enthalten orale Präparate gemäss US-Patentschrift 4 088 752 (H.R. Mühlemann und H. Schmid; Gaba AG), welche aus dem Salz einer Phosphorsäure und eines Amins, beispielsweise N,N,N'-tris-(2-Hydroxyäthyl)-N'-octadecyl-1,3-diaminopropandiorthophosphat, bestehen. Die Präparate können ausserdem Schleifmittel, Bindemittel, Anfeuchtungsmittel und oberflächenaktive Mittel enthalten.

Die metabolischen Effekte von niederen $SnF_2$-Konzentrationen gegen die Plaque gehen hauptsächlich auf die antibakterielle Wirkung der Zinn(II)-ionen zurück, welche wahrscheinlich Veränderungen der Zellmembrane der Bakterien bewirken. Es hat sich auch gezeigt, dass die Verminderung der Plaque unter der Einwirkung der Zinn(II)-ionen mit einem Rückgang der entzündlichen Zahnfleischerkrankung einhergeht.

In praktischer Hinsicht ist es aber ein bedeutender Nachteil, dass sich die Zinn(II)-salze, u.a. das Zinn(II)-fluorid, in mit Wasser verdünnten oralen Zubereitungen rasch zersetzen; dies gilt insbesondere für die niedrigen Konzentrationsbereiche und bei den im Mundbereich zulässigen pH-Werten (pH von ca. 4,5 bis 9,0), wie sie in den Mundwassern, Spüllösungen, Zahnpasten und anderen Mund- und Zahnpflegemitteln vorkommen. Frisch hergestellte Lösungen von Zinn(II)-fluorid oder von Zinn(II)-chlorid und einem Alkalifluorid zeigen innerhalb von einer bis 10 Minuten Opaleszenz und Trübung. Beim Stehenlassen bilden sich dann weisse Fällungen, welche sich während einer Zeitspanne von 3 bis 7 Tagen ständig vergrössern [Anon, Federal Register 39 (1974), 17245; Accepted Dental Therapeutics, 36. Aufl., Amer. Dent. Ass., Chicago (IL, USA) 1975]. Die Bildung von unlöslichen Ausfällungen wie das schwer lösliche Zinn(II)-hydroxid $Sn(OH)_2$ wird durch Hydrolyse und Oxidation der unbeständigen zweiwertigen Zinnionen verursacht. Die Bildung von Niederschlägen aus den Lösungen von Zinn(II)-fluorid oder -chlorid vermindert in demselben Ausmass die Schutzwirkung der Zinnionen auf die Zähne und ihre plaquehemmende Wirkung [I.L. Shannon, J. Southern Calif. State Dent. Ass. 32 (1964), 67]. Anhand von Beispielen wurde über die Einbusse an titrierbaren Zinn(II)-ionen in verdünnten (0,157%) $SnF_2$-Lösungen berichtet [J.K. Lim, J. Dent. Res. 49 (1970), 760]: Die Konzentration an Zinnionen fiel nach 1, 2, 5 und 7 Tagen um 8,1, 14,8, 36,3 bzw. 48,4%. Eine ähnliche Herabsetzung der Zinn(II)-ionen wird in alternden $SnF_2$-Zahnpasten ebenfalls beobachtet.

In der Herstellung von wasserhaltigen Mund- und Zahnpflegemitteln wurden bereits manche Versuche gemacht, um der Ausfällung der Zinnionen vorzubeugen. Die Methoden zur «Stabilisierung» des Zinn(II)-fluorids umfassen die extemporierte Auflösung in Wasser von festem Zinn(II)-fluorid aus harten Gelatinekapseln, wodurch die Herstellung eines frischen und wirksamen Mundspülwassers gewährleistet wird [P.E. Norris, J. Amer. Pharm. Ass. 20 (1959), 86]. Frische Spüllösungen können auch extemporiert, aus $SnF_2$-Tabletten, oder durch Verdünnung von hochkonzentrierten und stabileren 20%igen $SnF_2$-Lösungen hergestellt werden [I.L. Shannon u. Mitarb., J. Southern Calif. State Dent. Ass. 33 (1965), 520].

Eine lang anhaltende Beständigkeit ist durch Einarbeitung von Zinn(II)-fluorid in wasserfreies Glycerin erreicht worden [I.L. Shannon, Caries Res. 3 (1969), 339].

Wässrige Lösungen von Zinn(II)-fluorid mit Zusätzen von Natriumfluorid, Glycerin bzw. Weinsäure in geeigneten Konzentrationen können während einer beschränkten Zeitdauer bei Raum- und erhöhter Temperatur in klarem, niederschlagsfreiem und ästhetisch annehmbarem Zustand erhalten werden. Allerdings erweist sich die Konzentration an titrierbaren Zinn(II)-ionen als durch die Zusätze stark herabgesetzt [J.K. Lim, loc. cit. und J. Dent. Res. 50 (1971), 531].

Das mit der Unbeständigkeit der Zinn(II)-ionen einhergehende Problem ist auch von W.J. Griebstein (US-Patent Nr. 3 544 678) erkannt und die erforderliche Stabilisierung der oralen Kompositionen durch Bildung von wasserlöslichen Zinn(II)-komplexen mit komplexbildenden Anionen der Hydroxyethylnitrilodiessigsäure, m-Hydroxybenzoesäure, 1,2,3-Propantricarbonsäure und Itaconsäure vorgeschlagen worden. Nebst dem Anion und einer wasserlöslichen Zinn(II)-verbindung enthalten die Kompositionen ein wasserlösliches Fluorid in Form einer beliebigen anorganischen oder organischen Verbindung. Unter den zahlreichen Beispielen von organischen Aminhydrofluoriden befinden sich auch solche, die bereits 1958 von den vorliegenden Erfindern als Fluorionenquelle zur Kariesprophylaxe vorgeschlagen worden sind (US-Patent 3 083 143) und zum Teil unter die nachfolgenden Formeln I und II fallen.

Dieselben oder ähnliche langkettigen tertiären Amine mit einem $C_{12}$- bis $C_{18}$-Alkylrest besitzen übrigens antibakterielle und Zahnbelag verhindernde Eigenschaften. Deshalb sollen sie sowie auch entsprechend wirkende quaternäre Ammoniumverbindungen in Mundpflegemitteln verwendet werden. Die durch diese Verbindungen bewirkte Fleckenbildung der Zähne wird durch Zusatz von Mellithsäure oder Hexahydromellithsäure verhindert (DE-Offenlegungsschrift 2 755 847, FR-Patent 2 374 902). Die Mundpflegemittel in flüssiger Form enthalten als Träger gewöhnlich eine Wasser/Alkohol-Mischung. Als fakultative Komponenten kommen auch ein oberflächenaktiver Stoff und/oder eine Fluor liefernde Verbindung in Frage; Beispiele der letzteren sind anorganische Fluoride wie unter anderem Natriumfluorid, Zinn(II)-fluorid usw. Das Problem der Unbeständigkeit der Zinnfluoride in wässrigen Medien wird nicht gestreift.

Verbesserte Zahnpflegemittel enthalten Xylit, welches als Süssstoff und Feuchthaltemittel fungiert, und – nebst Wasser und einem Schleifmittel – auch ein Detergens (US-Pat. 3 932 604). In der Vielzahl der genannten Detergentien kommen ebenfalls Verbindungen der nachfolgenden Formel I und ihre Salze vor. Die Zahnpflegemittel können ferner Gummiharze, fluorhaltige Verbindungen und andere Additive enthalten; als Beispiele der fluorhaltigen Verbindungen werden wiederum die oben bereits erwähnten anorganischen Fluoride angeführt.

Gesamthaft betrachtet haben die zahlreichen Versuche zur Stabilisierung der wässrigen Lösungen von Zinn(II)-fluorid nur einen kleinen Teilerfolg erbracht, nämlich durch die «ex tempore»-Herstellung von Mundwassern. Bei den herkömmlichen Mund- und Zahnpflegemitteln, welche mindestens eine gewisse Lagerungsdauer unbeschadet ertragen sollen, ist das Problem hingegen – ausser durch die Bildung von Zinn(II)-komplexen nach W.J. Griebstein (siehe oben) – bisher ungelöst geblieben.

Es wurde nun überraschenderweise gefunden, dass die Zugabe von löslichen Aminsalzen zu wasserhaltigen Medien, welche Zinn(II)-fluorid, andere Zinn(II)-salze oder Mischungen von fluorfreien Zinnsalzen mit Fluoriden anderer Metalle, im allgemeinen in einer Zinnkonzentration von 0,005 bis 0,5 Gew.-%, enthalten, die Herstellung von beständigen, niederschlagsfreien Mund- und Zahnpflegemitteln ermöglicht.

Bei Verwendung von Zinn(II)-fluorid soll die Konzentration mit Vorteil im Bereich von 0,04 bis 0,6 Gew.-% $SnF_2$ liegen. Lösungen von Zinn(II)-fluorid werden durch Zugabe gewisser löslicher Aminsalze in einem Gewichtsverhältnis von Sn: Amin zwischen 1:10 und 10:1 stabilisiert; vorzugsweise werden die beiden Komponenten in äquimolarer Konzentration eingesetzt. Dadurch werden Gemische erhalten, welche während mehrerer Monate bei Raumtemperatur klar, frei von Zinniederschlägen bleiben und in welchen die Wirkungen von Zinn(II)-fluorid gegen die Plaque und gegen die Zahnfleischentzündung nicht beeinträchtigt werden.

Gegenstand der vorliegenden Erfindung sind also orale Kompositionen, welche in einem wasserhaltigen Medium Zinn(II)-fluorid oder eine Kombination eines fluorfreien Zinn(II)-salzes mit Natriumfluorid, Kaliumfluorid, Ammoniumfluorid oder einem Fluorid anderer Metalle und ein Hydrofluorid eines Amins der nachfolgend angegebenen Formeln in zur Stabilisierung der Zinnsalze ausreichender Menge enthalten.

Man wird also Zinn(II)-fluorid, vorzugsweise in einer Konzentration von 0,05 bis 0,5 Gew.-% Sn, verwenden oder die Zinnionen in Form eines fluorfreien Zinnsalzes wie Zinn(II)-chlorid, -bromid, -sulfat oder -tartrat zugeben, in diesem Fall aber in Verbindung mit Natriumfluorid, Kaliumfluorid, Ammoniumfluorid oder Fluoriden anderer Metalle.

Verwendet wird zur Stabilisierung ein Hydrofluorid eines Amins der Formel:

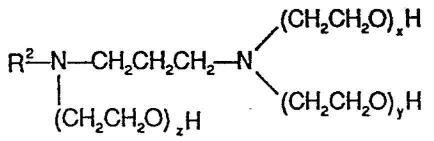

worin $R^2$ eine Fettalkylgruppe mit 7 bis 19 Kohlenstoffatomen und x, y und z jeweils eine ganze Zahl von 1 oder darüber bedeuten, oder der Formel:

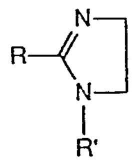

worin R eine Alkyl-, Alkenyl- oder Acylgruppe mit jeweils 7 bis 19 Kohlenstoffatomen oder eine Aralkylgruppe mit 8 bis 20 Kohlenstoffatomen und R′ ein Wasserstoffatom oder eine Alkyl-, Cycloalkyl-, Hydroxyalkyl-, Alkoxy-, Alkenyl-, Aralkyl- oder Acylgruppe bedeuten.

Geeignete Hydrofluoride solcher Amine sind u.a. in DE-PS 1 198 493 und US-PS 3 083 143 und 4 088 752 beschrieben worden.

In den Aminen der Formel bedeuten x, y und z vorzugsweise eine ganze Zahl von 1 bis 10. Ein typisches Beispiel davon ist das N,N,N′-tris-(2-Hydroxyäthyl)-N′-octadecyl-1,3-diaminopropan, welches in Form des Dihydrofluorides als Aminfluorid 297 bezeichnet und in AmF 297 abgekürzt wird [H.R. Mühlemann, Quintessenz 18 (1967), Hefte 5–8]; besonders bevorzugt ist das Dihydrofluorid des erwähnten 1,3-Diaminopropans.

Die unerwartete stabilisierende Wirkung der Aminsalze auf wässrige Lösungen von Zinn(II)-salzen wird im folgenden am Beispiel des Aminfluorids 297 gezeigt.

Versuch I

Es werden in destilliertem Wasser in Konzentrationen an Fluorid von 100, 250 und 10000 ppm (auf F berechnet) aufgelöst: Sn(II)-fluorid, das Aminfluorid 297 und die Kombination von $SnF_2$ mit AmF 297.

Die frisch hergestellten, klaren Lösungen werden im Heizschrank bei 48 °C gelagert und nach 1, 4 und 22 Stunden sowie auch nach 8 und 20 Tagen auf Trübungen oder/und Ausfällung geprüft. Aus der überstehenden Flüssigkeit der zentrifugierten Lösungen werden wiederholt Proben entnommen, welche zur Bestimmung des Zinngehaltes durch atomare Absorptionsspektrophotometrie und zur Bestimmung der Fluorion-Konzentration mittels der spezifischen Fluorion-Elektrode verwendet werden. Jede Versuchsreihe wurde dreimal wiederholt. Die Ergebnisse werden in Tabelle I wiedergegeben.

Versuch II

Die Stabilität von Aminfluorid 297 enthaltenden und im Heizschrank bei 48 °C bzw. bei Raumtemperatur (ca. 20 °C) gehaltenen $SnF_2$-Lösungen wurde auch während einer vier Wochen dauernden Zeitspanne durch Beobachtung der Niederschlagsbildung geprüft. Die Ergebnisse werden in der Tabelle II wiedergegeben.

Tabelle I

Zeitliches Auftreten einer Trübung (×) bzw. eines Niederschlages (××) im Heizschrank bei 48 °C (Mittelwerte von 3 Versuchen; ppm = Teile per Million)

| Wässrige Lösung von | Zinngehalt (Stunden) | | | | Trübung/Niederschlag (Stunden und *Tage*) | | | | | | Fluoriongehalt (Stunden) | | | |
|---|---|---|---|---|---|---|---|---|---|---|---|---|---|---|
| | 0 | 1 | 4 | 22 | 0 | 1 | 4 | 22 | *8* | *20* | 0 | 1 | 4 | 22 |
| (1) $SnF_2$ (F = 100 ppm, Sn = 312 ppm) | 289 | 281 | 245 | 118 | 0 | 0 | × | ×× | ×× | ×× | 89 | 96 | 85 | 85 |
| (2) AmF 297 (F = 100 ppm) | – | – | – | – | 0 | 0 | 0 | 0 | 0 | 0 | 106 | 105 | 100 | 100 |
| (3) $SnF_2$ + AmF 297 (1:1) (F = 100 ppm, Sn = 156 ppm) | 163 | 158 | 163 | 158 | 0 | 0 | 0 | 0 | 0 | 0 | 89 | 88 | 89 | 88 |
| (4) $SnF_2$ (F = 250 ppm, Sn = 780 ppm) | 750 | 730 | 676 | 530 | 0 | 0 | ×× | ×× | ×× | ×× | 240 | 240 | 228 | 228 |
| (5) AmF 297 (F = 250 ppm) | – | – | – | – | 0 | 0 | 0 | 0 | 0 | 0 | 253 | 253 | 252 | 250 |
| (6) $SnF_2$ + AmF 297 (1:1) (F = 250 ppm, Sn = 390 ppm) | 372 | 371 | 371 | 363 | 0 | 0 | 0 | 0 | 0 | 0 | 230 | 233 | 227 | 229 |
| (7) $SnF_2$ (F = 1000 ppm, Sn = 3123 ppm) | 3075 | 3025 | 2566 | 2515 | 0 | × | ×× | ×× | ×× | ×× | 871 | 868 | 798 | 836 |
| (8) AmF 297 (F = 1000 ppm) | – | – | – | – | 0 | 0 | 0 | 0 | 0 | 0 | 1051 | 1050 | 1057 | 1051 |
| (9) $SnF_2$ + AmF 297 (F = 1000 ppm), Sn = 1561 ppm) | 1590 | 1630 | 1600 | 1615 | 0 | 0 | 0 | 0 | (×) | (×) | 858 | 858 | 858 | 848 |

Tabelle II

Zeitliches Auftreten einer Trübung (×) bzw. eines Niederschlags (××) bei Raumtemperatur und im Heizschrank bei 48 °C (ppm = Teile per Million)

| Wässrige Lösung von | Konzentration an $F^-$ in ppm | pH | Temperatur in °C | Trübung/Niederschlag (*Stunden* und Tage) *2* | *20* | 2 | 3 | 7 | 14 | 21 | 28 |
|---|---|---|---|---|---|---|---|---|---|---|---|
| $SnF_2$ | 250 | 3,5 | ca. 20 | × | ×× | ×× | | | | | |
| | | | 48 | × | ×× | ×× | | | | | |
| $SnF_2$ + AmF 297 | 250 | 4,2 | ca. 20 | 0 | 0 | 0 | 0 | 0 | 0 | 0 | 0 |
| | | | 48 | 0 | 0 | 0 | 0 | 0 | 0 | 0 | 0 |
| $SnF_2$ | 1000 | 3,4 | ca. 20 | × | ×× | ×× | | | | | |
| | | | 48 | × | ×× | ×× | | | | | |
| $SnF_2$ + AmF 297 | 1000 | 3,9 | ca. 20 | 0 | 0 | 0 | 0 | 0 | 0 | 0 | 0 |
| | | | 48 | 0 | 0 | 0 | 0 | × | | | |

Die stabilisierende Wirkung von Aminfluorid 297 auf die wässrigen Lösungen von Zinn(II)-fluorid geht aus den Tabellen I und II eindrücklich hervor. 1 bis 4 Stunden alte $SnF_2$-Lösungen werden bereits trübe und bald danach bilden sich Niederschläge. Die Kombination des nicht ausfallenden Aminfluorids 297 mit Zinn(II)-fluorid in einer Konzentration an Fluor von 50 bis 100 ppm beugt der Bildung von Niederschlägen aus $SnF_2$ während bis zu 20 Tagen, und dies sogar bei einer Lagerungstemperatur von 48 °C, vor. Der Zinngehalt der überstehenden Flüssigkeit der zentrifugierten $SnF_2$-Lösungen, welche kein Amin enthielten, sank graduell während 22 Stunden; er blieb hingegen konstant in den Kombinationen $SnF_2$-AmF 297. Die Konzentrationen an Fluorionen in den überstehenden Flüssigkeiten wurden im Laufe der Zeit nicht wesentlich verändert (Tabelle I). Die stabilisierende Wirkung von AmF 297 auf $SnF_2$-Lösungen war in dem Heizschrank stärker ausgeprägt bei einer Fluorkonzentration von 250 ppm als bei der Fluorkonzentration von 1000 ppm. Bei Raumtemperatur konnten selbst nach 4 Wochen weder eine Trübung noch Ausfällungen beobachtet werden (Tabelle II).

Der Mechanismus, durch welchen die Zinnsalze stabilisiert werden, ist noch nicht aufgeklärt worden. Möglicherweise geht es auf die Bildung eines Chelates der Zinnionen durch aliphatische Diamine zurück, aber die Anwesenheit von Fluorionen in dem Zinn-Amin-System scheint auch für die Stabilisierung notwendig zu sein.

Die stabilisierende Wirkung des Aminfluorids 297 und im allgemeinen der Aminhydrofluoride auf wässrige Lösungen des Zinn(II)-fluorids ist deshalb von praktischer bzw. gewerblicher Bedeutung, weil sie nicht etwa mit einer Einbusse bei den Antiplaqueeigenschaften des Zinnfluorides oder der Aminsalze einhergeht. Dies wurde durch eine klinische Untersuchung von 4 Lösungen für Mundspülung gezeigt, welche mit Hilfe von 16 Hygienestudenten von 20 bis 26 Jahren durchgeführt wurde. Es wurden vier aufeinanderfolgende Spülperioden zu je 10 Tagen eingehalten; vor jeder dieser Perioden wurden die Zähne der Studenten einer beruflich durchgeführten Reinigung und Polierung unterworfen. Sie wurden dann dazu angewiesen, eine peinlich genaue Mundhygiene zu führen, um dadurch ein vollkommen gesundes Zahnfleisch zu gewährleisten. Die Freiwilligen wurden nach Zufall einer der vier folgenden Spüllösungen zugeteilt. Für jede der 10 Tage dauernden Spülperioden wurden die Lösungen nur einmal hergestellt und in undurchsichtige Kunststofflaschen abgefüllt.

Die Lösungen enthielten:

1. Chininchlorid (500 ppm) und ein Versüssungsmittel – Placebolösung, zur Kontrolle;
2. $SnF_2$ (250 ppm F) und das Versüssungsmittel;
3. N,N,N′-tris(2-Hydroxyäthyl)-N′-octadecyl-1,3-diaminopropandihydrofluorid (AmF 297; 250 ppm $F^-$) und das Versüssungsmittel; bzw.
4. $SnF_2$ (250 ppm $F^-$), AmF 297 (250 ppm $F^-$) und das Versüssungsmittel. In dieser Lösung betrug die Konzentration an Zinnionen und an der Aminkomponente von AmF 297 nur die Hälfte jener in den Lösungen 2 und 3.

Während der vier Spülperioden spülten die Studenten den Mund zweimal täglich während jeweils 30 Sekunden; sie hatten die Anweisung, vom Zähnebürsten und von anderen Massnahmen der Mundhygiene vollständig abzusehen. Am Ende einer jeden Spülperiode wurden die Zähne beruflich gereinigt und das Zähenbürsten wurde während 3 Tagen erlaubt, bis zum Beginn der nächsten, mit einer anderen Lösung durchzuführenden Spülperiode.

Jeder Freiwillige hat also mit den vier Lösungen entsprechend dem lateinischen Quadratschema gespült. Die Untersuchung wurde als Doppelblindvesuch durchgeführt.

Die Geschwindigkeit der Plaquebildung wurde durch den Plaque-Index bestimmt und durch normierte Farbphotographien belegt. Die metabolische Aktivität der Plaque wurde durch pH-Messungen abgeschätzt: Die am Ende jeder Spülperiode gesammelte Plaque wurde einer 10gew.-%igen Glucoselösung unterworfen und die Veränderung des pH-Wertes wurde nach 1, 10 und 20 Minuten gemessen. Die Zahnfleischentzündung

wurde mit dem Sulcus-Blutungs-Index und durch Messungen der Sulcus-Flüssigkeit (Exsudat) bestimmt. Die Ergebnisse werden in der Tabelle III wiedergegeben.

Tabelle III

Wirkung verschiedener Spüllösungen auf die Plaquebildung, die Zahnfleischblutung, das Zahnfleischexsudat und den pH-Wert der Plaque

(Mittelwerte ± durchschnittliche Abweichung)

| Diagnostischer Parameter | (1) Kontrolllösung | (2) $SnF_2$ (F = 250 ppm Sn = 312 ppm) | (3) AmF 297 (F = 250 ppm) | (4) $SnF_2$ + AmF 297 1:1 (F = 250 ppm Sn = 156 ppm) |
|---|---|---|---|---|
| Plaque-Index-Zunahme | 1,09 ± 0,52 | 1,14 ± 0,51 | 0,88 ± 0,56 | 0,57 ± 0,25*** |
| Plaque-pH-Abnahme 1 Min. nach Glucosebelastung | 0,67 ± 0,32 | 0,61 ± 0,39 | 0,30 ± 0,31** | 0,50 ± 0,38 |
| Sulcus Blutungs-Index | 0,35 ± 0,32 | 0,29 ± 0,33 | 0,27 ± 0,40 | 0,21 ± 0,40 |
| Sulcus-Flüssigkeit (Exsudat) in mm | 3,40 ± 4,70 | 3,09 ± 2,82 | 1,89 ± 2,4 | 1,34 ± 3,16* |

* Signifikanz gegenüber den Kontrollen < 0,05.
** Signifikanz gegenüber den Kontrollen < 0,01.
*** Signifikanz gegenüber den Kontrollen < 0,001.

Die Spülung mit gealterter $SnF_2$-Lösung oder mit Aminfluorid 297 allein zeigte keine signifikante Herabsetzung der Plaque und stand somit in klarem Gegensatz zur Spülung mit einer $SnF_2$ enthaltenden, mit dem Aminfluorid 297 stabilisierten Lösung; die photographische Diagnose bestätigte die sich in dem Plaque-Index widerspiegelnden Ergebnisse. Die bewirkte Herabsetzung der Plaquebildung war statistisch hoch signifikant; die beobachtete Potenzierung der Plaque-hemmenden Wirkung der beiden Komponenten war durchaus unerwartet, zumal in Anbetracht der in den Spüllösungen verwendeten niedrigen Konzentrationen an Zinnsalzen und an dem Aminhydrofluorid.

Die Fähigkeit der Zahnplaque zur Säurebildung aus Glucose (Glycolyse) war nur bei der das Aminhydrofluorid enthaltenden Spüllösung signifikant herabgesetzt.

Die Herabsetzung der Zahnfleischentzündung, durch den Sulcus-Blutungs-Index und die Messung der Zahnfleischexsudate bestimmt, erwies sich für das $SnF_2$ und AmF 297 enthaltende Mundwasser am stärksten, sie war für die $SnF_2$ oder AmF 297 allein enthaltenden Spüllösungen weniger ausgeprägt und statistisch nicht signifikant.

Insgesamt zeigte der Versuch mit jungen Erwachsenen, dass die Aminhydrofluoride wässrige Lösungen von Zinn(II)-fluorid effektiv stabilisieren und dass dabei die Antiplaquewirkung von $SnF_2$ und seine positive Wirkung gegen Zahnfleischentzündung voll erhalten bleiben und unter Umständen sogar eindrücklich potenziert werden.

Bei der praktischen Durchführung, d.h. bei der industriellen Herstellung von gebrauchsfertigen, alle üblichen Zutaten enthaltenden Mund- und Zahnpflegemitteln, wurde noch ausserdem gefunden, dass die Zugabe von nichtionogenen, wasserlöslichen Äthylenoxid-Addukten als Lösungsvermittler und Emulgator eine letzte Verbesserung in bezug auf die Stabilisierung der Zinnionen bewirkt. Es handelt sich dabei um Polyäther, insbesondere Alkylphenolpolylglykoläther und andere Produkte der Äthoxylierung von Fettsäuren, Fettsäureamiden, Fettaminen und Fettalkoholen.

Bekannte Beispiele dieser Stoffgruppe sind die unter dem Markennamen Cremophor® RH im Handel befindlichen Produkte der Firma Badische Anilin- und Sodafabrik, Ludwigshafen am Rhein (Bundesrepublik Deutschland). Diese Produkte werden durch Umsetzung von beispielsweise ca. 40 oder ca. 60 Mol Äthylenoxid mit 1 Mol hydriertem Ricinusöl hergestellt. Hauptbestandteile der Cremophor® RH-Produkte sind also Ester der hydrierten Ricinusölfettsäuren mit oxäthyliertem Glycerin. Daneben enthalten sie Polyglykolester der hydrierten Ricinuslöfettsäuren sowie freies oxäthyliertes Glycerin und höhere Polyäthylenglykole. Die sekundären OH-Gruppen der Oxystearinsäureester können zum Teil mit einem weiteren Oxystearinsäuremolekül verestert sein. Eine Oxäthylierung der sekundären OH-Gruppen der Oxystearinsäuremoleküle findet nur in sehr geringem Umfang statt.

Andere bekannte Beispiele von geeigneten Äthylenoxid-Addukten sind die Produkte Brij® und die Produkte Tween® der Firma ICI America Inc., Atlas Chemicals Div., Wilmington (DE, USA) sowie auch die Produkte Pluronic® der Firma Wyandotte Chemicals Co., Wyandotte (MI, USA). Bevorzugt werden darunter insbesondere Tween® 20, 40, 60 und 80 und Pluronic® F-68.

Es wurden parallele Versuchsreihen mit Mundspüllösungen durchgeführt, welche einen Gehalt von 0,1 Gew.-% des Emulgators Cremophor® RH enthielten und nach ihrer Herstellung 10 Tage lang bei Raumtemperatur bzw. bei 40°C gelagert wurden. Nach dieser Zeitspanne wurde die stabilisierende Wirkung durch visuelle Beurteilung des Aussehens ermittelt.

Die Grundzusammensetzung jeder Spüllösung war folgende:

| | |
|---|---|
| Ariavitblau 385, 0,2gew.-%ig (Farbstoff) | 1,0 g |
| Äthanol | 50,0 g |
| Aromastoffe | 1,2 g |
| Emulgator Cremophor® RH 40 oder 60 | 1,0 g |
| entionisiertes Wasser | bis auf 1000,0 g |

Der Gehalt an Wirkstoffen einer jeden Lösung geht aus folgender Tabelle hervor:

Tabelle IV

| Versuch 1 | Versuch 2 |
|---|---|
| $SnF_2$, allein | $SnF_2$, wie in Versuch 1 |
| 50 ppm $F^-$ | + AmF 297, jeweils |
| 100 ppm $F^-$ | 50 ppm $F^-$ |
| 250 ppm $F^-$ | |

Nach 10 Tagen zeigten die drei Lösungen von Versuch 1, ob bei Raumtemperatur oder bei 40 °C gelagert, einen leichten, aber deutlichen flockigen Niederschlag; die überstehenden Flüssigkeiten zeigten alle eine leichte Trübung. Mit zunehmender $SnF_2$-Konzentration machte sich eine stärkere Entfärbung der Lösung bemerkbar. Im Gegensatz dazu konnten sämtliche Lösungen von Versuch 2 als klar bezeichnet werden.

Es kann daraus ersehen werden, dass das Zinnfluorid durch den Emulgator allein nicht stabilisiert wird; hingegen bewirkt die Kombination des Aminsalzes und des Emulgators eine vollständige Stabilisierung des Zinn(II)-salzes in einer gebrauchsfertigen Mundspüllösung. Es konnten keine Unterschiede der Wirkung zwischen den bei Raumtemperatur und den bei 40 °C gelagerten Spüllösungen festgestellt werden.

Nach demselben Muster wie bei Tabelle IV angegeben, wurde der stabilisierende Einfluss des erwähnten Emulgators Cremophor® RH auf gebrauchsfertige Spüllösungen derselben Grundzusammensetzung untersucht, jedoch enthielten die Lösungen stets das Aminfluorid 297 und 10 Gew.-% Lycasin® 8055. Es handelt sich dabei um Hydrolyseprodukte von hydrierter Kartoffelstärke, welche durch die Firma Roquette Frères S.A., Lille (Frankreich), hergestellt werden; beispielsweise stellt das Produkt Lycasin® 8055 eine hochhydrierte Variante von Lycasin® dar. Der Zusatz von 0,1 Gew.-% Cremophor® RH erwies sich in jedem Fall positiv (klare, niederschlags- und trübungsfreie Lösung). Frisch hergestellte $SnF_2$-Lösungen zeigen nämlich innerhalb von einer bis 10 Minuten Opaleszenz und Trübung und danach weisse Fällungen (siehe Einleitung). In Kunststoffflaschen blieben die Lösungen in jedem Fall stabiler als in Glasflaschen. Die Lagerung bei 40 °C muss sich nicht immer negativ auswirken: Gegenüber der Lagerung bei Raumtemperatur wurden nämlich sowohl Verbesserungen als auch Verschlechterungen des Aussehens beobachtet.

In den folgenden Beispielen beziehen sich die Zahlenangaben auf Gewichtsteile; die %-Angaben beziehen sich ebenfalls auf Gewicht.

Bei den Mundwasserzubereitungen gemäss den Beispielen 1 bis 4 handelt es sich um gebrauchsfertige Spüllösungen, welche nicht verdünnt werden müssen.

Beispiel 1

Mundwasser

| | |
|---|---|
| N,N,N'-tris(2-Hydroxyäthyl)-N'-octadecyl-1,3-diaminopropan-dihydrofluorid | 0,14 (= 0,01% $F^-$) |
| Zinn(II)-fluorid | 0,04 (= 0,01% $F^-$) |
| Glycerin | 10,0 |
| Äthanol | 16,5 |
| Cremophor® RH 40 | 0,10 |
| Saccharin, Aromastoffe | 0,145 |
| Wasser | bis auf 100,0 |

Beispiel 2

Mundwasser

| | |
|---|---|
| N,N,N'-tris(2-Hydroxyäthyl)-N'-octadecyl-1,3-diaminopropan-dihydrofluorid | 0,2 |
| Zinn(II)-fluorid | 0,1 |
| Glycerin | 10,0 |
| Aromastoffe | 0,145 |
| Wasser | bis auf 100,0 |

Beispiel 3

Mundwasser

| | |
|---|---|
| N,N,N'-tris(2-Hydroxyäthyl)-N'-octadecyl-1,3-diaminopropan-dihydrochlorid | 0,50 |
| Zinn(II)-sulfat | 0,15 |
| Natriumfluorid | 0,05 |
| Lycasin® 8055 80%iger Sirup | 20,0 |
| Tween® 20 | 0,2 |
| Aromastoffe | 0,1 |
| Wasser | bis auf 100,0 |

Beispiel 4

Mundwasser

| | |
|---|---|
| Cetylamin-hydrofluorid | 0,025 |
| N,N,N'-tris(2-hydroxyäthyl)-N'-octadecyl-1,3-diaminopropan-dihydrofluorid | 0,3 |
| Zinn(II)-fluorid | 0,1 |
| Äthanol | 5,0 |
| Cremophor® RH 40 | 0,1 |
| Aromastoffe | 0,1 |
| Wasser | bis auf 100,0 |

Beispiel 5

Zahnpaste

| | |
|---|---|
| N,N,N'-tris(2-Hydroxyäthyl)-N'-octadecyl-1,3-diaminopropan-dihydrochlorid | 1,0 |
| Zinn(II)-fluorid | 0,2 |
| Natriumfluorid | 0,1 |
| Guargummi | 1,5 |
| Kieselsäure | 16,0 |
| Aromastoffe, Süssstoff | 1,5 |
| Wasser | bis auf 100,0 |

Beispiel 6

Dentalgelée

| | |
|---|---|
| Hydroxyäthylcellulose (Natrosol 250) | 3,0 |
| N,N,N'-tris(2-Hydroxyäthyl)-N'-octadecyl-1,3-diaminopropan-diyhdrofluorid | 1,25 |
| Zinn(II)-fluorid | 0,08 |
| Pluronic® F-68 | 1,5 |
| Aromastoffe, Farbstoffe | 0,5 |
| Wasser | bis auf 100,0 |

**Patentansprüche**

1. Orale Komposition, dadurch gekennzeichnet, dass sie in einem wasserhaltigen Medium Zinn(II)-fluorid oder eine Kombination eines fluorfreien Zinn(II)-salzes mit Natriumfluorid, Kaliumfluorid, Ammoniumfluorid oder einem Fluorid anderer Metalle und zu deren Stabilisierung ein Hydrofluorid eines Amins der Formel:

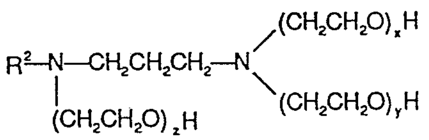

worin $R^2$ eine Fettalkylgruppe mit 7 bis 19 Kohlenstoffatomen und x, y und z jeweils eine ganze Zahl von 1 oder darüber bedeuten, oder der Formel:

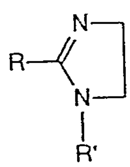

worin R eine Alkyl-, Alkenyl- oder Acylgruppe mit jeweils 7 bis 19 Kohlenstoffatomen oder eine Aralkylgruppe mit 8 bis 20 Kohlenstoffatomen und R' ein Wasserstoffatom oder eine Alkyl-, Cycloalkyl-, Hydroxyalkyl-, Alkoxy-, Alkenyl-, Aralkyl- oder Acylgruppe bedeuten, und gegebenenfalls ausserdem als einziges weiteres Stabilisierungsmittel und zugleich als Lösungsvermittler und Emulgator nichtionogene wasserlösliche Ethylenoxid-Addukte enthält.

2. Komposition nach Anspruch 1, dadurch gekennzeichnet, dass die gegebenenfalls vorhandenen Ethylenoxid-Addukte Alkylphenolpolyglykolether oder andere Produkte der Ethoxylierung von Fettsäuren, Fettsäureamiden, Fettaminen oder Fettalkoholen sind.

3. Komposition nach Anspruch 1 oder 2, dadurch gekennzeichnet, dass das Hydrofluorid ein solches des N,N,N'-tris-(2-Hydroxyethyl)-N'-octadecyl-1,3-diaminopropans ist.

4. Komposition nach einem der Ansprüche 1 bis 3, dadurch gekennzeichnet, dass sie in der Form von Zahnpasten, Gelées, Mundwassern, Mundsprays, Prophylaxepasten oder Lösungen zur Lokalapplikation vorliegt.

**Claims**

1. An oral composition which comprises, in a water containing medium, tin (II) fluoride or a combination of a fluorine-free tin (II) salt with sodium fluoride, potassium fluoride, ammonium fluoride or a fluoride of other metals and for stabilization thereof a hydrofluoride of an amine of the formula:

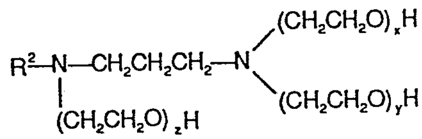

wherein $R^2$ denotes a fatty alkyl group with 7 to 19 carbon atoms and x, y and z each denote an integer from 1 onwards, or of the formula:

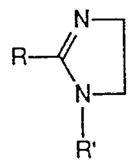

wherein R denotes an alkyl, alkenyl or acyl group with in each case 7 to 19 carbon atoms or an aralkyl group with 8 to 20 carbon atoms and R' denotes a hydrogen atom or an alkyl, cycloalkyl, hydroxyalkyl, alkoxy, alkenyl, aralkyl or acyl group, and optionally, in addition, as a single further stabilizing agent and at the same time as a solubilizing agent and emulsifier, nonionic water-soluble addition products of ethylene oxide.

2. A composition according to claim 1, wherein the optional addition products of ethylene oxide are alkylphenol polyglycol ethers or other products resulting from the oxyethylation of fatty acids, fatty acid amides, fatty amines or fatty alcohols.

3. A composition according to claim 1 or 2, wherein the hydrofluoride is such one of N,N,N'-tris-(2-hydroxyethyl)-N'-octadecyl-1,3-diaminopropane.

4. A composition according to any one of claims 1 to 3, which is in the form of toothpastes, dental gels, mouthwashes, mouth sprays, prophylaxis pastes or solutions for topical application.

**Revendications**

1. Composition orale, caractérisée en ce qu'elle contient, dans un milieu aqueux, du fluorure d'étain(II) ou une association d'un sel d'étain(II) exempt de fluor avec du fluorure de sodium, du fluorure de potassium, du fluorure d'ammonium ou un fluorure d'autres métaux et, pour leur stabilisation, un fluorhydrate d'une amine de formule:

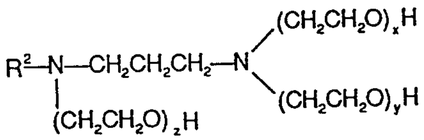

dans laquelle $R^2$ représente un groupe alcoyle gras important de 7 à 19 atomes de carbone et x, y et z représentent chacun un nombre entier de 1 ou plus, ou de formule:

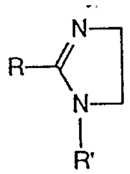

dans laquelle R représente un groupe alcoyle, alcényle ou acyle comportant chacun de 7 à 19 atomes de carbone ou un groupe aralcoyle comportant de 8 à 20 atomes de carbone et R' représente un atome d'hydrogène ou un groupe alcoyle, cycloalcoyle, hydroxy alcoyle, alcoxy, alcényle, aralkyle ou acyle et cas échéant, en outre, comme seul autre agent stabilisant ainsi que comme agent solubilisant et émulsifiant des produits d'addition d'oxyde d'éthylène, non ionogènes, solubles dans l'eau.

2. Composition selon la revendication 1, caractérisée en ce que les produits d'addition d'oxyde d'éthylène éventuellement présents sont des éthers d'alcoylphénol polyglycol ou d'autres produits de l'éthoxylation des acides gras, des amides gras, des amines grasses ou des alcools gras.

3. Composition selon la revendication 1 ou 2, caractérisée en ce que le fluorhydrate est un fluorhydrate du N,N,N'-tris-(2-hydroxyéthyl)-N'-octadécyl-1,3-diaminopropane.

4. Composition selon l'une des revendications 1 à 3, caractérisée en ce qu'elle se présente sous la forme de pâtes dentrifrices, de gels, de bains de bouche, d'aérosols pour la bouche, de pâtes prophylactiques ou de solutions pour application locale.